# EUROPÄISCHE PATENTSCHRIFT

(11) **EP 1 717 578 B1**
(45) Veröffentlichungstag und Bekanntmachung des Hinweises auf die Patenterteilung: **06.02.2008**
(21) Anmeldenummer: 06007566.0
(22) Anmeldetag: 11.04.2006
(51) Int. Cl.: G01N 27/411

(54) **Eintauchmesssonde für Metallschmelzen**
Immersion probe for metal melts
Sonde de mesure d'immersion pour des métaux en fusion

(30) Priorität: 26.04.2005 DE 102005019665
(43) Veröffentlichungstag der Anmeldung: 02.11.2006
(73) Patentinhaber: Heraeus Electro-Nite International N.V., 3530 Houthalen (BE)
(72) Erfinder: Gerits, Erik, 3600 Genk (BE)
(74) Vertreter: Kühn, Hans-Christian

(56) Entgegenhaltungen:
- WO-A-02/06804
- DE-A1- 10 203 121
- US-A- 4 906 349
- US-A- 5 656 143
- US-A1- 2004 084 328
- US-A1- 2004 173 473

## Beschreibung

Die Erfindung betrifft eine Eintauchmesssonde, insbesondere Einwurfmesssonde für Metallschmelzen mit einem Messkopf, an dem mindestens ein Sensorträger mit mindestens einem Sensor angeordnet ist, wobei der Sensorträger an oder in einer Öffnung des Messkopfes gehalten ist.

Derartige Messsonden sind beispielsweise aus DE 198 49 433 C1 bekannt. Gattungsgemässe Messsonden sind auch aus WO 02/06804 A bekannt. Einwurfmesssonden werden aus einer gewissen Höhe aus einer Halterung heraus in eine Metallschmelze geworfen. Der an einem Ende eines Trägerrohres gehalterte Messkopf ist in der Regel aus Stahl gebildet, um die nötige Masse zum Durchschlagen der auf einer Stahl- oder Eisenschmelze aufliegenden Schlackeschicht aufzuweisen. Innerhalb des Trägerrohres ist ein Signalkabel aufgewickelt, welches mit einer Messeinrichtung verbunden ist und welches sich während des Herabfallens in die Metallschmelze aus dem Trägerrohr heraus abwickelt. In dem Messkopf ist ein Sensorträger angeordnet mit mindestens einem Sensor, beispielsweise einem Temperatursensor oder einem elektrochemischen Sensor zum Messen des Sauerstoffgehalts der Metallschmelze. Beim Durchdringen der auf einer Stahlschmelze aufliegenden Schlackeschicht kann am Messkopf Schlacke haften bleiben. Diese am Messkopf, ggf. an der Schutzkappe für die Sensoren, anhaftende Schlacke kann die Messung der Eigenschaften der Stahlschmelze beeinflussen und zu Messfehlem führen. Elektrochemische Sensoren benötigen neben dem Kontakt ihres Festelektrolytmaterials mit der Metallschmelze auch einen sogenannten Badkontakt zwischen ihrer sogenannten Gegenelektrode und der Metallschmelze. Der Badkontakt wird häufig außerhalb der Messsonde über Signalleitungen zur Schmelze hin realisiert (DE 30 21 949 C2), oder, wie beispielsweise in DE 195 31 661 A1 oder DE 196 52 596 A1 beschrieben, als zusätzliches Bauteil, das wiederum entsprechend zu schützen ist. Diese Veröffentlichungen betreffen allerdings keine Einwurfmesssonden und sind den besonderen mechanischen Belastungen (insbesondere Stoßbelastungen) solcher Sonden nicht ausgesetzt.

Aus DE 102 03 121 A1 ist eine Sensoreinheit bekannt, bei der ein Sensorträger an einem Messkopf angeordnet ist, wobei der Sensorträger einen Sensor aufweist und wobei der Messkopf mit Teilen des Sensors elektrisch kontaktiert ist. Ein ähnlicher Sensor ist aus EP 363 616 A2 bekannt. Aus DE 101 03 701 C1 ist des weiteren ein Festelektrolytröhrchen bekannt, welches in einer Messeinrichtung zur Bestimmung von Sauerstoff in Metallschmelzen Verwendung findet. Das Festelektrolytröhrchen ist auf seinem rückseitigen Ende mit einer Kappe rastend verschlossen.

Der Erfindung liegt die Aufgabe zugrunde, eine einfach aufgebaute und zuverlässig funktionierende Eintauchmesssonde, insbesondere Einwurfmesssonde, bereit zu stellen.

Die Aufgabe wird durch eine Eintauchmesssonde gemäß Anspruch 1 gelöst. Vorteilhafte Ausgestaltungen sind in den Unteransprüchen angegeben.

Dadurch, dass mindestens ein Teil des Messkopfes mit einer Gegenelektrode eines am Messkopf angeordneten elektrochemischen Sensors elektrisch kontaktiert ist und einen Badkontakt des elektrochemischen Sensors bildet, entsteht ein zuverlässiger Badkontakt durch einfache und kurze Kontaktwege und eine große Badkontaktoberfläche. Die Anordnung ist sehr stabil und hält den mechanischen Belastungen beim Einwerfen der Sonde in eine Stahlschmelze stand. Vorzugsweise ist der Messkopf aus Metall gebildet. Insbesondere ist es zweckmäßig, dass der Sensorträger rastend mit dem Messkopf verbunden ist, um eine einfache Montage zu gewährleisten. Es ist am Sensorträger, vorzugsweise an dessen im Inneren des Messkopfes angeordneten Ende, ein erstes Rastelement angeordnet, das mit einem am Messkopf angeordneten zweiten Rastelement in Eingriff steht. Vorteilhaft ist es weiterhin, dass ein Kontaktelement am Sensorträger, vorzugsweise an dessen im Inneren des Messkopfes angeordneten Ende, angeordnet ist, welches die Gegenelektrode mit dem Messkopf elektrisch leitend verbindet. Es ist zweckmäßig, dass das erste Rastelement an dem Kontaktelement angeordnet ist.

Nachfolgend wird ein Ausführungsbeispiel der Erfindung an Hand einer Zeichnung näher erläutert. In der Zeichnung zeigt:
- Fig. 1: einen Querschnitt durch eine Ausführungsform des Messkopfes sowie eine Draufsicht,
- Fig. 2: ein Kontaktelement.

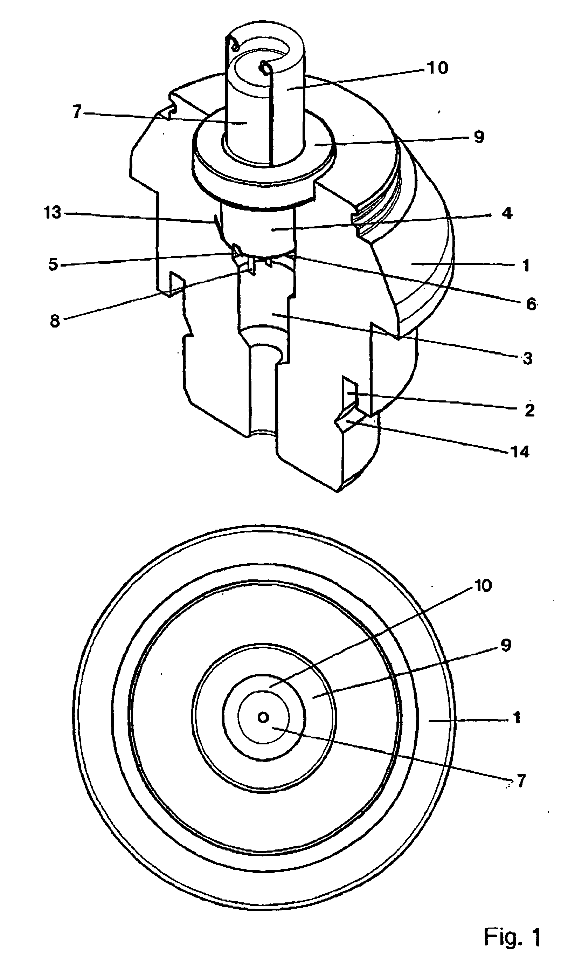

In Figur 1 ist der Messkopf 1 einer Einwurfmesssonde dargestellt. Der Messkopf 1 ist üblicherweise an einem Ende eines Trägerrohres, welches aus Pappe gebildet sein kann, gehaltert. Die Ringnut 2 oder die Ringnut 14 des Messkopfes 1 können dazu beitragen. Das Trägerrohr ist aus Gründen der Übersichtlichkeit in der Zeichnung nicht dargestellt.
Der Messkopf 1 besteht aus Stahl. Er weist eine zentrische Längsbohrung 3 auf. Im Eintauchende des Messkopfes 1 ist in der Bohrung 3 ein Sensorträger 4 gehaltert. Der Sensorträger 4 weist an seiner dem Eintauchende abgewandten Rückseite eine oder mehrere Widerhaken 5 als erstes Rastelement auf, die beim Einschieben des Sensorträgers 4 in die Bohrung 3 in eine umlaufende Nut 6 als zweites Rastelement einrasten und den Sensorträger 4 in der Bohrung 3 fixieren. Der Sensorträger 4 weist unter einer Schutzkappe 7 einen oder mehrere Sensoren auf, beispielsweise ein Thermoelement und/oder einen elektrochemischen Sensor zur Bestimmung eines Gasgehaltes der Stahlschmelze, zu deren Analyse die Einwurfmesssonde eingesetzt wird. Der Sensor ist über Kontakte 8 an der Rückseite des Sensorträgers 4 mit Signalleitungen verbunden, die durch die Bohrung 3 hindurch mit einem Messgerät oder einem Computer verbunden sind.

Der in der Bohrung 3 des Messkopfes 1 angeordnete Sensorträger 4 weist einen Ring 9 auf, der aus Gießereisand gebildet ist. Es ist auch möglich, den kompletten Sensorträger 4 aus Gießereisand auszubilden. Zumindest der Ring 9 kann auch aus Pappe oder einem ähnlichen verbrennbaren oder Gas enthaltenden porösen Material gebildet sein. Der Sensor des Sensorträgers ist mit einer Schutzkappe 7 abdeckt, die wiederum mit einer Pappkappe 10 bedeckt ist. Sowohl die Pappkappe 10 als auch die Schutzkappe 7 werden beim Eintritt in die Stahlschmelze vernichtet, so dass der Sensor mit der Stahlschmelze in Berührung gelangt. Bei Durchtauchen der auf der Stahlschmelze aufliegenden Schlackeschicht bzw. beim Eintauchen in die Stahlschmelze heizt sich der Messkopf sehr stark bis zur Temperatur der Schmelze auf. Dabei erfährt das in den Poren des Gießereisandes vorhandene Gas eine schnelle Volumenvergrößerung, so dass es sehr schnell aus den Ring 9 austritt und dabei eventuell an dem Sensorträger 4 oder der Schutzkappe 7 anhaftende Schlacke wegreißt. Dadurch beeinträchtigt die Schlacke die unmittelbare Umgebung des Sensors nicht, so dass die Messgenauigkeit erhöht wird.

Der Sensorträger 4 kann aber auch aus Stahl gebildet sein. Der Messkopf 1 kann prinzipiell auch ohne ein Trägerrohr verwendet werden.

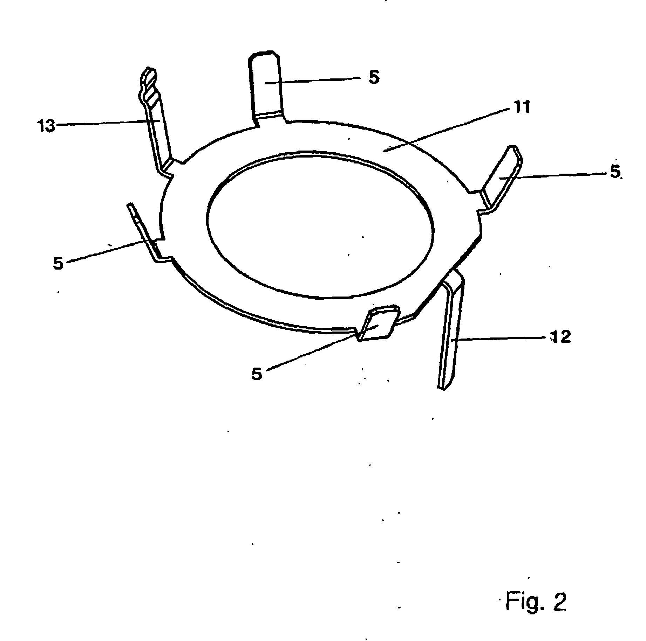

Ein am Sensorträger 4 angeordnetes elektrochemisches Element benötigt zu seiner Funktionsfähigkeit einen sogenannten Badkontakt. Dieser Badkontakt wird zweckmäßigerweise dadurch hergestellt, dass der entsprechende, aus der Unterseite des Sensorträgers 4 herausgeführte Kontakt des elektrochemischen Elementes (Gegenelektrode) über ein Kontaktelement 11 (dargestellt in Figur 2) direkt mit den metallischen Messkopf 1 elektrisch verbunden ist, so dass der Körper des Messkopfes 1 selbst den elektrischen Kontakt zur Metallschmelze herstellt. Das Kontaktelement 11 ist im wesentlichen als Metallring ausgebildet und an der im Innern des Messkopfes 1 angeordneten Stirnseite des Sensorträgers 4 angeordnet. Eine Kontaktlasche 12 dient der Kontaktierung mit der Gegenelektrode und eine weitere Kontaktlasche 13 dient der Kontaktierung mit dem Messkopf 1. Dadurch ist zum einen eine einfache Montage des Sensorträgers 4 in dem Messkopf 1 gewährleistet, da beide lediglich ineinander gesteckt werden müssen und dann rastend miteinander verbunden sind. Zum anderen entsteht ein zuverlässiger Badkontakt durch einfache und kurze Kontaktwege und eine große Badkontaktoberfläche. Die Anordnung ist sehr stabil und hält den mechanischen Belastungen beim Einwerfen der Sonde in eine Stahlschmelze stand.

## Patentansprüche

1. Eintauchmesssonde, insbesondere Einwurfmesssonde für Metallschmelzen mit einem Messkopf (1), an dem mindestens ein Sensorträger (4) mit mindestens einem Sensor angeordnet ist, wobei der Sensorträger an oder in einer Öffnung des Messkopfes gehalten ist, wobei mindestens ein Teil des Messkopfes mit einer Gegenelektrode eines am Messkopf angeordneten elektrochemischen Sensors elektrisch kontaktiert ist und einen Badkontakt des elektrochemischen Sensors bildet, **dadurch gekennzeichnet, dass** am Sensorträger ein erstes Rastelement angeordnet ist, das mit einem am Messkopf angeordneten zweiten Rastelement (6) Eingriff steht, dass ein Kontaktelement (11) am Sensorträger (4), angeordnet ist, welches die Gegenelektrode mit dem Messkopf elektrisch leitend verbindet und dass das erste Rastelement (5) und das Kontaktelement (11) aus einem gemeinsamen Werkstück bestehen.

2. Eintauchmesssonde nach Anspruch 1, **dadurch gekennzeichnet, dass** der Messkopf aus Metall gebildet ist.

3. Eintauchmesssonde nach Anspruch 1 oder 2, **dadurch gekennzeichnet, dass** der Sensorträger rastend mit dem Messkopf verbunden ist.

4. Eintauchmesssonde nach einem der Ansprüche 1 bis 3, **dadurch gekennzeichnet, dass** das erste Rastelement am im Inneren des Messkopfes angeordneten Ende des Sensorträgers angeordnet ist.

## Claims

1. Immersion measuring probe, in particular a drop-in measuring probe for metal melts, comprising a measuring head (1), on which at least one sensor carrier (4) with at least one sensor is arranged, wherein the sensor carrier is held on or in an opening of the measuring head, at least a part of the measuring head being in electrical contact with a counter-electrode of an electrochemical sensor arranged at the measuring head and forming a bath contact of the electrochemical sensor, **characterised in that** a first locking element (5) is arranged on the sensor carrier and engages with a second locking element (6) arranged on the measuring head, **in that** a contact element (11) is arranged on the sensor carrier (4), which contact element connects the counter-electrode directly to the measuring head in an electrically conductive manner and **in that** the first locking element (5) and the contact element (11) consist of a common workpiece.

2. Immersion measuring probe according to claim 1, **characterised in that** the measuring head is formed from metal.

3. Immersion measuring probe according to claim 1 or 2, **characterised in that** the sensor carrier is in locking connection with the measuring head.

4. Immersion measuring probe according to any one of claims 1 to 3, **characterised in that** the first locking element is arranged on the end of the sensor carrier arranged in the interior of the measuring head.

## Revendications

1. Sonde de mesure par immersion, notamment sonde de mesure par immersion largable, pour des bains de métal en fusion, comprenant une tête de mesure (1) sur laquelle est agencé au moins un support de capteur (4) avec au moins un capteur, le support de capteur étant maintenu dans ou au niveau d'une ouverture de la tête de mesure, au moins une partie de la tête de mesure étant contactée électriquement avec une électrode conjuguée d'un capteur électrochimique agencé sur la tête de mesure et formant un contact de bain du capteur électrochimique, **caractérisée en ce que** sur le support de capteur est agencé un premier élément d'encliquetage (5) qui est en prise avec un deuxième élément d'encliquetage (6) agencé sur la tête de mesure, **en ce qu'**un élément de contact (11) est agencé sur le support de capteur (4) et relie directement, sur le plan électrique, l'électrode conjuguée avec la tête de mesure, et **en ce que** le premier élément d'encliquetage (5) et l'élément de contact (11) sont réalisés par une pièce commune.

2. Sonde de mesure par immersion selon la revendication 1, **caractérisée en ce que** la tête de mesure est réalisée en métal.

3. Sonde de mesure par immersion selon la revendication 1 ou 2, **caractérisée en ce que** le support de capteur est relié ou assemblé par encliquetage à la tête de mesure.

4. Sonde de mesure par immersion selon l'une des revendications 1 à 3, **caractérisée en ce que** le premier élément d'encliquetage est agencé à l'extrémité du support de capteur, disposée à l'intérieur de la tête de mesure.
